# EUROPEAN PATENT SPECIFICATION

(11) **EP 3 719 462 B1**
(45) Date of publication and mention of the grant of the patent: **15.12.2021**
(21) Application number: 20162045.7
(22) Date of filing: 10.03.2020
(51) Int. Cl.: G01K 1/06, G01K 1/08, G01K 5/02, G01K 5/22, G01K 13/00

(54) **A CASE FOR A BODY TEMPERATURE-MEASURING THERMOMETER HAVING A REGION WITH A MAGNIFYING GLASS EFFECT FOR READING THE GRADUATED SCALE OF THE THERMOMETER**
GEHÄUSE FÜR EIN KÖRPERTEMPERATURMESSTHERMOMETER MIT EINEM BEREICH MIT LUPENEFFEKT ZUM ABLESEN DER SKALA DES THERMOMETERS
ÉTUI POUR UN THERMOMÈTRE DE MESURE DE LA TEMPÉRATURE CORPORELLE COMPORTANT UNE ZONE À EFFET DE LOUPE POUR LA LECTURE DE L'ÉCHELLE GRADUÉE DU THERMOMÈTRE

(30) Priority: 12.03.2019 IT 201900000817 U
(43) Date of publication of application: 07.10.2020
(73) Proprietor: Pikdare-Società per Azioni, 22070 Casnate con Bernate CO (IT)
(72) Inventor: REZZONICO, Fabio, 22070 Casnate con Bernate (IT); RUIZ BRAVO, Alejandro José, 22100 Como (IT); SPOLETINI, Silvia, 22070 Casnate con Bernate (IT)
(74) Representative: Perani & Partners S.p.A.

(56) References cited:
- CN-A- 106 595 882
- US-A- 3 052 158

## Description

The present invention relates to a case for a thermometer for measuring the temperature of a sick individual, especially for domestic use, but without excluding professional use by physicians or nurses.

The case is of the type that comprises a compartment extending along a major longitudinal axis, with one open end equipped with a removable opening/closing member, through which the thermometer is introduced into the compartment.

One longitudinally delimiting wall of the compartment is known to be possibly formed with at least one transparent zone providing a magnifying glass effect, to facilitate reading of the graduated scale of the thermometer when the latter is stored in the compartment of the case. A case of this type is disclosed e.g. by CN 106 595 882 A and US 3,052,158 A.

According to the prior art, when the case has a compartment with a rectangular cross section, it has a wall whose outer surface forms a base surface of the case for the latter to lie on a table or a support surface.

Since this base wall is normally opposite and parallel to the wall in which the section or zone with the magnifying glass effect is formed, such arrangement has been found, during use, to hinder reading of the graduated scale of the thermometer, when the latter is within the case.

That is, with such arrangement the user is required to look at the transparent zone from above when the case lies on a support surface, such as a table beside the bed, unless he/she picks it up and brings it close to his/her face.

This constitutes a drawback especially if the user is sick and bedridden.

Therefore, the object of the present invention is to provide a case structure for a body temperature thermometer, equipped with transparent magnifying glass zone, affording facilitated reading of the graduated scale of the thermometer, when it is within the case and the latter lies on a support surface, without requiring it to be held on one hand to look for an appropriate position for reading.

This and other objects, as more clearly explained hereinbelow, are fulfilled by a case for a thermometer for measuring the body temperature of a sick individual comprising a compartment extending along a major longitudinal axis with one open end, for receiving the thermometer into the compartment, said compartment having at least one transparent zone with a magnifying glass effect for reading of the graduated scale of the thermometer when the latter is stored in the compartment of the case, said transparent zone forming part of a wall of the compartment of the case, said compartment further comprising a wall whose outer surface forms a base surface of the case, characterized in that said transparent zone, with the magnifying glass effect is formed on a surface that is contiguous to said base surface of the case, and forms an angle therewith, whose vertex is placed on the side of the case from which the graduated scale of the thermometer is read through said transparent zone.

The invention will be now described in greater detail with reference to one embodiment thereof, given by way of illustration and without limitation, and depicted in the annexed drawings, in which:

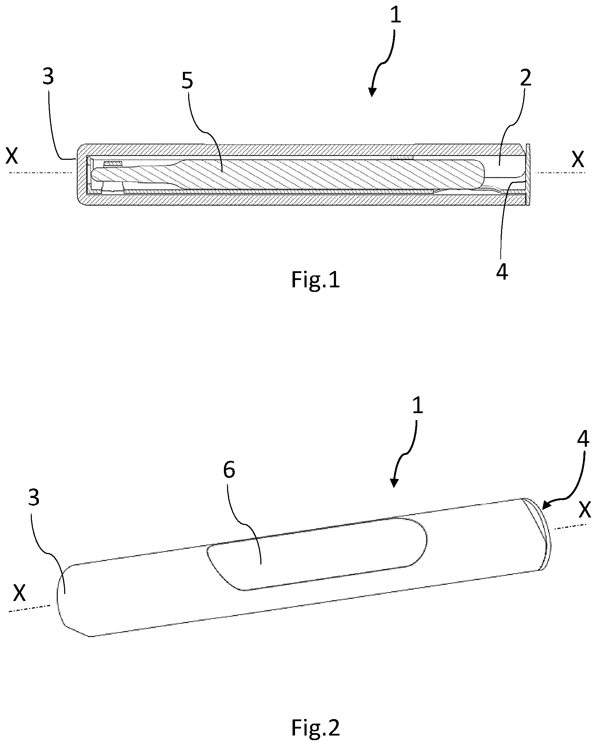

- Figure 1 shows a cross-sectional view of the case according to a first embodiment of the invention, with the thermometer therein;
- Figure 2 shows a perspective view of the case of Figure 1, without the thermometer therein;

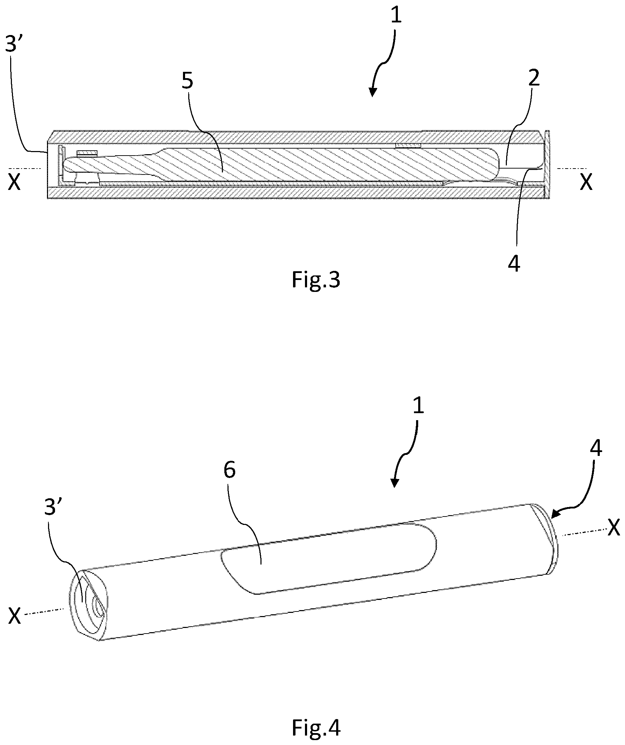

- Figure 3 shows a cross-sectional view of the case according to a second embodiment of the invention, with the thermometer therein;
- Figure 4 shows a perspective view of the case of Figure 3, without the thermometer therein;

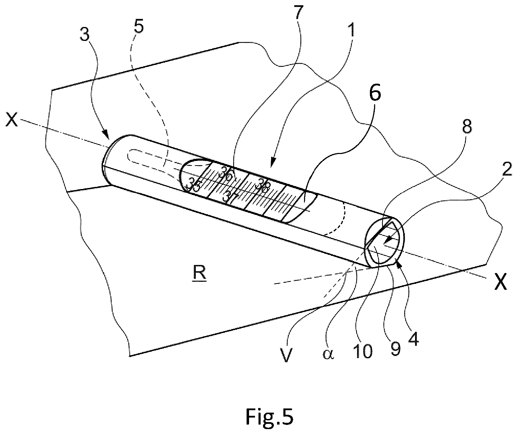

- Figure 5 shows a perspective schematic view of the case of Figure 1, as taken from its open end with the thermometer therein, the case lying on a support surface.

Referring to the aforementioned figures, numeral 1 generally designates the case of the invention. It comprises a compartment 2, extending along a major longitudinal axis X-X.

The compartment 2 has one open end 4, which is nevertheless equipped with a closure member, e.g. in the form of a cap or lid, not shown because it is known in the art, which may be used to close the case, once the body temperature thermometer 5 has been introduced therein.

According to a first embodiment, the compartment 2 comprises a closed end 3 opposite to the open end 4 along the longitudinal axis X-X.

According to a second embodiment, alternative to the first embodiment, the compartment 2 comprises an additional open end 3' opposite to the open end 4 along the longitudinal axis X-X.

The aforementioned compartment 2 has at least one zone 6 that is transparent and provides a magnifying glass effect, for facilitated reading of the graduated scale 7 of the thermometer 5, when the latter is within the compartment of the case.

The transparent zone 6 forms a part of a wall 8 of the compartment of the case 1, whereas another wall 9 of the compartment 2 forms with its outer surface a base surface of the case 1 for the latter to be laid, for instance, on a support surface, generally referenced R, which may be for example a table beside a bed.

In order to make sure that the graduated scale 7 of the thermometer is placed in front of the transparent zone 6, said compartment 2 of the case has a base surface 10 therein, on which the thermometer or a case thereof may be laid, which is opposite to the surface 8 of said transparent zone.

According to the invention, the wall 8, or at least the surface in which the transparent zone 6 with the magnifying glass effect is formed, and the base wall 9 of the case, form an angle, referenced α, whose vertex V is located on the side from which the graduated scale of the thermometer is read through the transparent zone 6.

With the aforementioned arrangement, the provision of the angle α facilitates reading of the graduated scale of the thermometer, when the case lies on the support surface R, even by a patient that lies in a bed, obviously as long as said transparent zone 7 faces him/her.

In particular, the angle α is an angle that ranges from 30 to 90° with respect to the plane of the wall 9 and, preferably, from 50° to 60°.

The materials that form the case 1 may be of various natures, including plastic materials, as long as they are compatible with the function to be accomplished.

## Claims

1. A case (1) for a thermometer (5) for measuring the body temperature of a sick individual, comprising a compartment (2) extending along a major longitudinal axis (X-X) with one open end (4), for receiving the thermometer (5) into the compartment (2), said compartment (2) having at least one transparent zone (6) with a magnifying glass effect for reading of the graduated scale (7) of the thermometer (5) when the latter is within the compartment (2) of the case, said transparent zone (6) forming part of a wall (8) of the compartment of the case, said compartment (2) further comprising a wall (9) whose outer surface forms a base surface of the case, **characterized in that** said transparent zone (6) with the magnifying glass effect forms a surface (8) that intersects said base surface (9) of the case, and forms an angle (α) therewith, whose vertex (V) is placed on the side of the case (1) from which the graduated scale of the thermometer is read through said transparent zone.

2. A case as claimed in claim 1, **characterized in that** said angle (α) ranges from 30 to 90 with respect to said base surface (9) of the case.

3. A case as claimed in claims 1 and 2, **characterized in that** said angle (α) ranges from 50° to 60° with respect to said base surface (9) of the case.

4. A case as claimed in any of claims 1 to 3, **characterized in that** said compartment (2) of the case has a base surface (10) therein, on which the thermometer or a case thereof may be laid, which is opposite to the surface of said transparent zone (6) with the magnifying glass effect,

5. A case as claimed in any of claims 1 to 4, **characterized in that** said wall (8) of the compartment (2) of the case with said transparent area (6) with the magnifying glass effect, also forms a surface oriented at an angle (α) with respect to said base surface of the case.

6. A case as claimed in any of claims 1 to 5, **characterized in that** the compartment (2) comprises a closed end (3) opposite to the open end (4) along the longitudinal axis (X-X).

7. A case as claimed in any of claims 1 to 5, **characterized in that** the compartment (2) comprises an additional open end (3') opposite to the open end (4) along the longitudinal axis (X-X).

## Patentansprüche

1. Gehäuse (1) für ein Thermometer (5) zum Messen der Körpertemperatur einer kranken Person, mit einem sich entlang einer Hauptlängsachse (X-X) erstreckenden Fach (2) mit einem offenen Ende (4) zur Aufnahme des Thermometers (5) in dem Fach (2), wobei das Fach (2) mindestens eine transparente Zone (6) mit Lupeneffekt zum Ablesen der Skala (7) des Thermometers (5) aufweist, wenn dieses sich in dem Fach (2) des Gehäuses befindet, wobei die transparente Zone (6) Teil einer Wand (8) des Fachs des Gehäuses bildet, wobei das Fach (2) außerdem eine Wand (9) umfasst, deren Außenfläche eine Basisfläche des Gehäuses bildet, **dadurch gekennzeichnet, dass** die transparente Zone (6) mit Lupeneffekt eine Fläche (8) bildet, die sich mit der Basisfläche (9) des Gehäuses schneidet und mit dieser einen Winkel (a) bildet, dessen Scheitelpunkt (V) auf der Seite des Gehäuses (1) angeordnet ist, von der aus die Skala des Thermometers durch die transparente Zone abgelesen wird.

2. Gehäuse nach Anspruch 1, **dadurch gekennzeichnet, dass** der Winkel (a) im Bereich von 30 bis 90 in Bezug auf die Basisfläche (9) des Gehäuses liegt.

3. Gehäuse nach Anspruch 1 und 2, **dadurch gekennzeichnet, dass** der Winkel (a) im Bereich von 50° bis 60° in Bezug auf die Basisfläche (9) des Gehäuses liegt.

4. Gehäuse nach einem der Ansprüche 1 bis 3, **dadurch gekennzeichnet, dass** das Fach (2) des Gehäuses eine Basisfläche (10) darin aufweist, auf die das Thermometer oder ein Gehäuse davon gelegt werden kann, die der Oberfläche der transparenten Zone (6) mit dem Lupeneffekt gegenüberliegt.

5. Gehäuse nach einem der Ansprüche 1 bis 4, **dadurch gekennzeichnet, dass** die Wand (8) des Fachs (2) des Gehäuses mit dem transparenten Bereich (6) mit dem Lupeneffekt auch eine Fläche bildet, die in einem Winkel (a) in Bezug auf die Basisoberfläche des Gehäuses ausgerichtet ist.

6. Gehäuse nach einem der Ansprüche 1 bis 5, **dadurch gekennzeichnet, dass** das Fach (2) ein geschlossenes Ende (3) umfasst, das dem offenen Ende (4) entlang der Längsachse (X-X) gegenüberliegt.

7. Gehäuse nach einem der Ansprüche 1 bis 5, **dadurch gekennzeichnet, dass** das Fach (2) ein zusätzliches offenes Ende (3') umfasst, das dem offenen Ende (4) entlang der Längsachse (X-X) gegenüberliegt.

## Revendications

1. Boîtier (1) pour un thermomètre (5) destiné à la mesure de la température corporelle d'un individu malade, comprenant un compartiment (2) s'étendant le long d'un axe longitudinal principal (X-X) avec une extrémité ouverte (4), pour recevoir le thermomètre (5) dans le compartiment (2), ledit compartiment (2) ayant au moins une zone transparente (6) avec un effet de loupe pour la lecture de l'échelle graduée (7) du thermomètre (5) lorsque celui-ci est à l'intérieur du compartiment (2) du boîtier, ladite zone transparente (6) faisant partie d'une paroi (8) du compartiment du boîtier, ledit compartiment (2) comprenant en outre une paroi (9) dont la surface externe forme une surface de base du boîtier, **caractérisé en ce que** ladite zone transparente (6) avec l'effet de loupe forme une surface (8) qui coupe ladite surface de base (9) du boîtier, et forme un angle (a) avec celle-ci, dont le sommet (V) est placé sur le côté du boîtier (1) à partir duquel l'échelle graduée du thermomètre est lue à travers ladite zone transparente.

2. Boîtier selon la revendication 1, **caractérisé en ce que** ledit angle (a) est compris entre 30 et 90 ° par rapport à ladite surface de base (9) du boîtier.

3. Boîtier selon les revendications 1 et 2, **caractérisé en ce que** ledit angle (a) est compris entre 50° et 60° par rapport à ladite surface de base (9) du boîtier.

4. Boîtier selon l'une quelconque des revendications 1 à 3, **caractérisé en ce que** ledit compartiment (2) du boîtier a une surface de base (10) dans celui-ci, sur laquelle le thermomètre ou un boîtier de celui-ci peut être posé, qui est opposée à la surface de ladite zone transparente (6) avec l'effet de loupe.

5. Boîtier selon l'une quelconque des revendications 1 à 4, **caractérisé en ce que** ladite paroi (8) du compartiment (2) du boîtier avec ladite zone transparente (6) avec l'effet de loupe, forme également une surface orientée selon un angle (a) par rapport à ladite surface de base du boîtier.

6. Boîtier selon l'une quelconque des revendications 1 à 5, **caractérisé en ce que** le compartiment (2) comprend une extrémité fermée (3) opposée à l'extrémité ouverte (4) le long de l'axe longitudinal (X-X).

7. Boîtier selon l'une quelconque des revendications 1 à 5, **caractérisé en ce que** le compartiment (2) comprend une extrémité ouverte supplémentaire (3') opposée à l'extrémité ouverte (4) le long de l'axe longitudinal (X-X).
